Europäisches Patentamt

European Patent Office

Office européen des brevets

(19)

(11) Veröffentlichungsnummer: **0 357 561**
**A2**

(12) **EUROPÄISCHE PATENTANMELDUNG**

(21) Anmeldenummer: **89810636.4**

(22) Anmeldetag: **28.08.89**

(51) Int. Cl.5: **B 01 F 17/44**
**C 11 D 1/06, C 11 D 3/20**

(30) Priorität: **30.08.88 DE 3829315**

(43) Veröffentlichungstag der Anmeldung:
**07.03.90 Patentblatt 90/10**

(84) Benannte Vertragsstaaten:
**AT BE CH DE ES FR GB IT LI NL SE**

(71) Anmelder: **SANDOZ AG**
**Lichtstrasse 35**
**CH-4002 Basel (CH)**

(84) Benannte Vertragsstaaten:
**BE CH ES FR GB IT LI NL SE**

(71) Anmelder: **SANDOZ-PATENT-GMBH**
**Humboldtstrasse 3**
**D-7850 Lörrach (DE)**

(84) Benannte Vertragsstaaten: **DE**

(71) Anmelder: **SANDOZ-ERFINDUNGEN**
**Verwaltungsgesellschaft m.b.H.**
**Brunner Strasse 59**
**A-1235 Wien (AT)**

(84) Benannte Vertragsstaaten: **AT**

(72) Erfinder: **Chavannes, Jean-Pierre**
**2 Rue de Montreux**
**F-68300 Saint-Louis (FR)**

(54) **Stabile Lösungen von carboxymethylierten Aethylenoxidaddukten.**

(57) Stabile, konzentrierte wäsrige Lösungen von carboxymethylierten Aethylenoxidaddukten von Fettalkoholen oder Alkylphenolen werden erhalten durch Verwendung einer Kombination von Lösungsvermittlern aus mindestens zwei Komponenten gleicher oder verschiedener Kategorie der Gruppen
(A) Glykol- oder Diglykol-monoäther
(B) Alkylendiol ,
wobei eine der Komponenten durch einen niederen Alkohol ersetzt werden kann oder ein solcher Alkohol als weitere Komponente anwesend ist. Die Lösungen sind temperaturstabil und gut giessbar.

EP 0 357 561 A2

**Beschreibung**

## STABILE LOESUNGEN VON CARBOXYMETHYLIERTEN AETHYLENOXIDADDUKTEN

Carboxymethylierte Aethylenoxidaddukte von Fettalkoholen oder Alkylphenolen sind als hervorragende Tenside mit einer vielfältigen Anwendung in Waschmitteln, Kosmetika, usw. bekannt. Je nach Konstitution fallen diese Produkte in fester oder flüssiger Form an, aber auch die flüssige Form ist als solche nicht einfach zu handhaben, weil die Verdünnung mit Wasser problematisch ist. Deshalb werden diese Produkte normalerweise als stark verdünnte wässrige Lösung angeboten, die als solche weiterverarbeitet werden muss. Diese Feststellung trifft besonders für solche Addukte zu, die aus Fettalkoholen mit $C_{8-18}$Fettrest, weiter bevorzugt mit einem durchschnittlichen $C_{12-15}$Fettrest, oder aus Isooctyl- oder Nonylphenol als lipophilem Baustein gebildet sind, und die daneben relativ kurze Aethylenoxidketten aufweisen, insbesondere zwischen 4 und 5 Aethylenoxideinheiten. Durch das ausgewogene Gleichgewicht zwischen lipophilem und hydrophilem Molekülbestandteil zusammen mit ca. 1 Carboxymethylgruppe besitzen diese Addukte ausgezeichnete tenside Eigenschaften.

Es besteht jedoch ein Bedarf an stabilen, konzentrierten wässrigen Lösungen von derartigen carboxymethylierten Aethylenoxidaddukten, die ohne weiteres mit Wasser verdünnt werden können und daher für die Herstellung von Waschmitteln, Kosmetika, usw. durch Mischung mit anderen Komponenten optimal geeignet sind. Bislang hat sich gezeigt, dass jeder Versuch, eine solche konzentrierte Lösung in stabiler Form herzustellen, fehlschlägt, weil entweder hochviskose Pasten entstehen, die nicht mehr giessbar sind, oder Lösungen erhalten werden, die nach kurzer Zeit einen Bodensatz bilden oder sich in zwei Phasen trennen. Auch der Zusatz von Lösungsmitteln wie Alkoholen oder auch Glykolen ist in den meisten Fällen ungeeignet, das Problem der dargelegten Aufgabe zufriedenstellend zu lösen; ausserdem ist er mit dem Nachteil verbunden, dass die meisten dieser Lösungsvermittler einen tiefen Flammpunkt aufweisen und daher Lösungen liefern, die ein Sicherheitsrisiko darstellen.

Es wurde nun gefunden, dass stabile, konzentrierte wässrige Lösungen von carboxymethylierten Aethylenoxidaddukten von Fettalkoholen oder Alkylphenolen hergestellt werden können, wenn als Lösungsvermittler eine Kombination von mindestens zwei Komponenten gleicher oder verschiedener Kategorie der folgenden Gruppen (A) und (B)

(A) ein Glykol- oder Diglykol-monoäther der Formeln

$R-O-CH_2CH_2OH$ (1)

$R-O-CH_2CH_2O-CH_2CH_2OH$ (2)

worin R für $C_{1-4}$Alkyl steht,

(B) ein Diol der Formel

HO-Alk-OH

worin Alk lineares oder verzweigtes $C_{3-6}$Alkylen bedeutet, eingesetzt wird.

In einer Verbindung (1) oder (2) kann R als $C_{1-4}$Alkyl linear oder verzweigt sein; insbesondere bevorzugt steht R für Butyl.

Die Alkylengruppe des Diols der Gruppe (B) enthält bevorzugt 6 C-Atome und ist insbesondere bevorzugt verzweigt.

Die Lösungen gemäss obiger Zusammensetzung mit Addukt, Wasser und Kombination an Lösungsvermittler enthalten mindestens 33 Gewichtsprozente an Addukt als Aktivstoff (bezogen auf 100% Gesamtgewicht der Lösung); sie sind trotzdem leicht giessbar, d.h. sie weisen eine Brookfield-Viskosität bis maximal 80'000, vorzugsweise von höchstens 20'000 mPa s (mit Spindel Nr. 7 bei 50 rpm) auf; ausserdem sind sie stabil, weder bei 0°C noch bei 50°C trennen sich Bestandteile aus der Lösung ab.

Durch Verwendung einfacher Alkohole wie n-Propylalkohol oder Isobutylalkohol allein lassen sich zwar ähnliche Ergebnisse erreichen, doch haben diese Alkohole einen derart tiefen Flammpunkt, dass ihr Einsatz als alleiniges Lösungsmittel mit einem gewissen Risiko verbunden ist.

Bei der erfindungsgemäss einzusetzenden Kombination an Lösungsvermittlern handelt es sich um eine Mischung aus mindestens zwei Komponenten der Gruppen (A) und (B) wie oben definiert, bevorzugt um eine Mischung von mindestens je einer Komponente von jeder der Gruppen (A) und (B), die zusammen vorzugsweise in einer Menge von 3 bis 7% bezogen auf 100% Gesamtgewicht der Lösung vorliegen. Besonders vorteilhaft wird ein Gemisch aus Butoxyäthanol bzw. Butoxyäthoxyäthanol (der Gruppe A) mit 2-Methyl-2,4-diolpentan (der Gruppe B) eingesetzt, und zwar vorteilhaft 2 bis 4% der ersten Komponente zusammen mit 1 bis 4% der zweiten Komponente. Bevorzugte Lösungen gemäss der Erfindung enthalten diese Bestandteile im Gewichtsverhältnis 2:1 bis 1:2, weiter bevorzugt im Verhältnis 3:2.

Des weiteren ist es auch möglich, für die Kombination an Lösungsvermittlern als eine der Komponenten oder auch als zusätzliche Komponente einen niederen Alkohol $R_1OH$, worin $R_1$ für lineares oder verzweigtes $C_{3-6}$Alkyl steht, zu verwenden. Im Hinblick auf das zu berücksichtigende Flammpunktrisiko ist dabei die Menge an verwendeter Komponente $R_1OH$ nach oben begrenzt, sie liegt bevorzugt bei maximal 3% bezogen auf 100% Gesamtgewicht der Lösung. Die Stabilität derartiger Lösungen ist gut, die Viskosität liegt im bevorzugten Bereich bis höchstens 20'000 mPa s.

In bevorzugten Lösungen sind die carboxymethylierten Aethylenoxidaddukte als Aktivstoff in einer Menge von 50-60% bezogen auf 100% Gesamtgewicht der Lösung enthalten.

Die nachstehenden Beispiele sollen die Erfindung näher erläutern, diese jedoch nicht einschränken.

Beispiel 1

Ein carboxymethyliertes Aethylenoxidaddukt der durchschnittlichen Zusammensetzung $C_{12-15}H_{25-31}$-O-$(CH_2CH_2O)_{4,5}$-$(CH_2COONa)_{0,8}$ wird als 93%ige wässrige Paste eingesetzt und zusammen mit einer entsprechenden Menge Wasser, 3 Teilen Butyldiglykol (= Butoxyäthoxyäthanol) und 2 Teilen Hexylenglykol (= 2-Methyl-2,4-diolpentan) gerührt, bis eine homogene Lösung vorliegt, deren Zusammensetzung in der folgenden Tabelle ausgewiesen ist. Die erhaltene Lösung ist einwandfrei giessbar und sowohl bei 0°C als auch bei 50°C unbegrenzt stabil; sie hat die Viskosität 1300 mPa s.

Weitere Lösungen lassen sich in analoger Weise wie oben beschrieben herstellen, indem man das in Beispiel 1 verwendete carboxymethylierte Aethylenoxidaddukt mit der entsprechenden Menge Wasser und dem erf indungsgemässen Lösungsvermittlergemisch rührt. Sie sind in der Tabelle als Beispiele 2-10 aufgeführt. Alle diese Lösungen sind problemlos giessbar, zeichnen sich durch Temperaturstabilität aus und haben eine Brookfield-Viskosität im bevorzugten Bereich (wie angegeben).

In der Tabelle erfolgen die Mengenangaben in Gewichtsprozenten bezogen auf 100% Gesamtgewicht der Lösung. Ausserdem ist für jede Lösung die Viskosität angegeben. Alle Viskositäten wurden auf einem Brookfield-Viskometer gemessen.

Butylglykol ist Butoxyäthanol;
Methyldiglykol ist Methoxyäthoxyäthanol.

Tabelle

Mengenangaben in %

Beispiele 1-10

| Beispiel Nr. | 1 | 2 | 3 | 4 | 5 | 6 | 7 | 8 | 9 | 10 |
|---|---|---|---|---|---|---|---|---|---|---|
| Addukt (umgerechnet auf wasserfreien Aktivstoff) | 50 | 50 | 50 | 50 | 50 | 50 | 50 | 50 | 50 | 50 |
| Wasser | 45 | 45 | 45 | 44 | 43,6 | 44 | 45 | 45 | 44 | 45 |
| Butylgylkol | - | 3 | - | 3 | 4 | - | 3 | 3 | - | - |
| Butyldiglykol | 3 | - | - | - | - | 3 | - | - | 5 | 2 |
| Hexylenglykol | 2 | 2 | 3 | 3 | - | 3 | - | - | 1 | 3 |
| Methyldiglykol | - | - | - | - | - | - | 2 | - | - | - |
| Isobutylalkohol | - | - | 2 | - | 2,4 | - | - | - | - | - |
| n-Propylalkohol | - | - | - | - | - | - | - | 2 | - | - |
| Total | 100% | 100% | 100% | 100% | 100% | 100% | 100% | 100% | 100% | 100% |
| Brookfield-Viskosität mPa s | 1300 | 2100 | 2000 | 1800 | 1800 | 1100 | 14000 | 2500 | 4500 | 3200 |

In den Beispielen 3, 5 und 8 wird als zweite Komponente ein niederer Alkohol mitverwendet. Der kritische Flammpunkt dieser Lösungen liegt
für Beispiel 3 bei 69°C,
für Beispiel 5 bei 63°C und
für Beispiel 8 bei 56°C,
was vom Risikostandpunkt aus gesehen vertretbar ist.
Die Flammpunktsbestimmung erfolgte auf einer Apparatur Abel Penski Automatic (APA-3).

## Patentansprüche

1. Stabile, konzentrierte wässrige Lösungen von carboxymethylierten Aethylenoxidaddukten von Fettalkoholen oder Alkylphenolen, dadurch gekennzeichnet, dass diese Lösungen neben den Addukten und Wasser als Lösungsvermittler eine Kombination von mindestens zwei Komponenten gleicher oder verschiedener Kategorie der folgenden Gruppen (A) und (B)
(A) ein Glykol- oder Diglykol-monoäther der Formeln
$R-O-CH_2CH_2OH$ (1)
$R-O-CH_2CH_2O-CH_2CH_2OH$ (2)
worin R für $C_{1-4}$Alkyl steht,
(B) ein Diol der Formel
$HO-Alk-OH$
worin Alk lineares oder verzweigtes $C_{3-6}$Alkylen bedeutet,
enthalten.

2. Lösungen gemäss Anspruch 1, dadurch gekennzeichnet, dass sie auf 100% Gesamtgewicht eine Menge von 3 bis 7% der in Patentanspruch 1 angeführten Kombination an Lösungsvermittlern enthalten.

3. Lösungen gemäss Anspruch 1 oder 2, dadurch gekennzeichnet, dass sie als Lösungsvermittler eine Kombination aus einer Komponente der Gruppe (A) zusammen mit einer Komponente der Gruppe (B) enthalten.

4. Lösungen gemäss Anspruch 3, dadurch gekennzeichnet, dass als Komponente der Gruppe (A) eine Verbindung (1) oder (2), worin R für Butyl steht, und als Komponente der Gruppe (B) Hexylenglykol der Formel

$$(CH_3)_2 \underset{OH}{C}CH_2 \underset{OH}{C}HCH_3$$

verwendet wird.

5. Lösungen gemäss Anspruch 4, dadurch gekennzeichnet, dass sie die beiden Komponenten der Gruppen (A) und (B) im Verhältnis 2:1 bis 1:2 enthalten.

6. Lösungen gemäss Anspruch 1 oder 2, dadurch gekennzeichnet, dass sie mindestens 33% Aktivsubstanz an carboxymethyliertem Aethylenoxidaddukt von Fettalkoholen oder Alkylphenolen enthalten.

7. Lösungen gemäss Anspruch 1 oder 2, dadurch gekennzeichnet, dass für die Kombination an Lösungsvermittler als eine der Komponenten oder auch als zusätzliche Komponente ein niederer Alkohol $R_1OH$, worin $R_1$ für lineares oder verzweigtes $C_{3-6}$Alkyl steht, eingesetzt wird.

8. Lösungen gemäss Anspruch 7, dadurch gekennzeichnet, dass die Menge an einer Komponente $R_1OH$ maximal 3% bezogen auf 100% Gesamtgewicht der Lösung beträgt.

9. Lösungen gemäss einem der Ansprüche 1 bis 8, dadurch gekennzeichnet, dass sie eine Brookfield-Viskosität von höchstens 20'000 mPa s (Spindel Nr. 7, 50 rpm) aufweisen.